# EUROPEAN PATENT SPECIFICATION

(11) **EP 1 202 728 B1**
(45) Date of publication and mention of the grant of the patent: **19.10.2005**
(21) Application number: 00952813.4
(22) Date of filing: 03.08.2000
(51) Int. Cl.: A61K 31/28, A61K 31/44, A61K 31/555, A61K 33/24, A61P 29/00

(54) **PEROXOVANADIUM COMPOUNDS AS PROTEIN TYROSINE PHOSPHATASE (PTP) INHIBITORS: THEIR INHIBITORY EFFECTS ON ANGIOGENESIS, RESTENOSIS AND THE PRODUCTION OF ENDOTHELINS.**
PEROXOVANADIUMVERBINDUNGEN ALS PROTEIN TYROSIN PHOSPHATASE (PTP) INHIBITORE: IHRE INHIBIERENDE WIRKUNG AUF ANGIOGENESIS, RESTENOSIS UND DIE PRODUKTION VON ENDOTHELINEN.
COMPOSES DE PEROXOVANADIUM COMME INHIBITEURS DE LA PROTEINE TYROSINE PHOSPHATASE (PTP): LEURS EFFETS INHIBITEURS SUR L'ANGIOGENESE, LA RESTENOSE ET LA PRODUCTION D'ENDOTHELINES.

(30) Priority: 12.08.1999 CA 2280249
(43) Date of publication of application: 08.05.2002
(60) Divisional application: 05004368.6
(73) Proprietor: Université Laval, Québec, Québec G1K 7P4 (CA)
(72) Inventor: FAURE, Robert, Québec G1S 3T3 (CA); SAVARD, Pierre, Québec G6V 4X5 (CA); DOILLON, Charles, Québec G2A 3L2 (CA); BATTISTINI, Bruno, Joseph, St-Nicholas, Québec G7A 4X9 (CA); OLIVIER, Martin, Québec G1T 1B6 (CA); POSNER, Barry, Québec H3Z 1Z2 (CA)
(74) Representative: Vaillant, Jeanne
(86) International application number: PCT/CA2000/000898
(87) International publication number: WO 2001/012180

(56) References cited:
- WO-A-00/57860
- M.OLIVIER E.A.: "PV-mediated protection against Leishmaniasis" JOURNAL OF LEUCOCYTE BIOLOGY, no. 132, 1999, page 31 XP000982208
- G.R.WILLSKY E.A.: "Pharmacology and toxicology of oxovanadium species: oxovanadium pharmacology " AMERICAN CHEMICAL SOCIETY. ABSTRACTS OF PAPER. AT THE NATIONAL MEETING, AMERICAN CHEMICAL SOCIETY, WASHINGTON, DC, US, vol. 711, 1998, pages 278-296, XP000965226
- M.OLIVIER E.A.: "Modulation of Interferon-gamma-induced macrophage activation by phosphotyrosine phosphatases inhibition" JOURNAL OF BIOLOGICAL CHEMISTRY, vol. 273, no. 22, 1998, pages 13944-13949, XP002160782
- A.MORINVILLE E.A.: "From Vanadis to Atropos: vanadium compounds as pharmacoligical tools in cell death signalling" TRENDS IN PHARMACOLOGICAL SCIENCES TIPS, vol. 19, 1998, pages 452-460, XP004142799
- O.J.D'CRUZ E.A.: "Oxovanadium (4) complexes are potent anti-cancer agents against human germ cell tumors" AMERICAN CHEMICAL SOCIETY. ABSTRACTS OF PAPER. AT THE NATIONAL MEETING, AMERICAN CHEMICAL SOCIETY, WASHINGTON, DC,US, vol. 217, no. 1/02, 1999, page 239 XP000965660

## Description

### FIELD OF THE INVENTION

This invention relates to the use of peroxovanadium compounds, such as potassium bisperoxo(1,10-phenanthroline)oxovanadate [bpV(phen)], potassium bisperoxo(pyridine-2-carboxylato)oxovanadate [bpV(pic)] and potassium bisperoxo(2,2'-bipyridyl) oxovanadate [bpV(bipy)], for preventing angiogenesis, restenosis and the production of endothelins, and as adjuvants for vaccination.

### BACKGROUND OF THE INVENTION

### A) PEROXOVANADIUM COMPOUNDS:

Synthetic peroxovanadium (pV) compounds are structurally versatile molecules which are potent inhibitors of phosphotyrosyl phosphatases (PTPs) (1). These compounds contain one oxo ligand, one or two peroxo groups coordinated to vanadium, and one ancillary ligand, and are stable in aqueous solution at physiological pH when shielded from light. The mechanisms underlying the PTPs' inhibitory potency and the specificity of these peroxanion compounds have been characterized. Compared to known inhibitors of PTPs such as orthovanadate, molybdate, tungstate and zinc, they have the ability to irreversibly oxidize an essential, conserved cysteine residue located in the catalytic domain (2). The possibility to manipulate the ancillary ligands may be important in regulating potency and specificity (1, 3). A reversible arrest of the proliferation of transformed cells by two pV compounds, bpV(phen) and bpV(pic), has been reported (4). The administration of pV compounds to mice has cured aggressive infections induced by a protozoan parasite (*Leishmaniasis*) (5).

### B) ANGIOGENESIS:

Almost all tissues and organs develop a vascular network which provides cells with nutrients and oxygen and enables the elimination of metabolic waste. Once formed, the vascular network is a stable system with a slow rate of cellular turn over (6) and yet, the endothelium can become one of the most rapidly proliferating of all cell types when stimulated (7). Indeed, the formation of new blood vessels can cause serious physiological complications. For example, while cornea and cartilage are avascular in healthy situations, several diseases involving these tissues are complicated by the massive arrival of blood vessels. Eye angiogenic diseases include neovascular glaucoma, retrolental fibroplasia, macular degeneration and neovascularization of corneal grafts. Joint angiogenic diseases include rheumatoid arthritis and arthrosis. Psoriasis, a chronic condition of the skin, also exhibits hypervascularization at the surface of the skin. Finally, solid tumor growth is critically dependent upon the formation of new blood vessels to progress locally and spread all over the body (8,9). The maintenance of existing blood vessels also requires the regulation of cell replication and/or differentiation. For example, acute and chronic pathological processes, such as atherosclerosis, post-angioplastic restenosis and hypertension, involve the proliferation of different cellular components of mature blood vessels (endothelial cells, smooth muscle cells, myocytes and fibroblasts).

Several factors, including certain peptides and proteins, can induce a vascular response *in vivo*. They are endogenous substances, such as EGF/TGF-α (Epidermal Growth Factor/Transforming Growth Factor-alpha), TGF-β (Transforming Growth Factor-beta), TNF-α (Tumor Necrosis Factor-alpha), angiogenin, prostaglandine E₂, and monobutyrine (10-15). However, these factors have almost no mitogenic effect on endothelial cells in culture (TGF-α, EGF, angiogenine, prostaglandine E₂, monobutyrine) and, paradoxically, inhibit their growth (TGF-β, TNF-α) (10-17). Their angiogenic action is thus indirect, depending for the most part upon the inflammatory response (18). Inflammatory cells produce some factors, such as aFGF (acidic Fibroblast Growth Factor), bFGF (basic Fibroblast Growth Factor), PDGF (Platelet-Derived Growth Factor), and VEGF (Vascular Endothelial Growth Factor), which are capable of stimulating the proliferation of endothelial cells *in vitro* and angiogenesis *in vivo* (19-28).

The angiogenic process, as currently understood, can be summarized as follows: a cell activated by a mutation, lack of oxygen, etc, releases angiogenic molecules (29-34) that attract inflammatory and endothelial cells and promote their proliferation. Following the binding of leukocytes to vascular endothelial cells, the latter reorganize the protein arrangements on their membranes to activate the angiogenic process (35, 36). During migration to the target tissue, inflammatory cells release substances that intensify the angiogenic effect (37, 38). Activated vascular endothelial cells respond to the angiogenic signal by secreting proteases which digest blood vessel walls to enable migration toward the target tissue (39-41). Several protein fragments produced by the digestion of the blood-vessel walls intensify the proliferative and migratory activity of the endothelial cells (42-44). Finally, the endothelial cells rearrange their adhesive membrane proteins to generate the formation of capillary tubes.

Angiogenesis is thus a complex process consisting of several critical cellular events (45-47), among which the following may be readily identified:
- binding of leukocytes to endothelial cells and induction;
- migration of inflammatory cells to the target tissue;
- regression of the pericytes of the existing vascular system;
- dissolution of blood-vessel walls by proteases;
- endothelial cell migration;
- endothelial cell proliferation;
- endothelial cell differentiation and arrangement into a tubular shape;
- formation of the capillary network;
- anastomosis; and
- initiation of blood flow.

Agents that are known to induce the proliferation of endothelial cells include sodium orthovanadate (48). The mechanism by which this agent induces an invasive phenotype in capillary endothelial cells is not clearly understood. However, the effects of vanadate on cultured cells are similar, in many respects, to those elicited by PMA (49), bFGF (50), and certain retroviral transforming proteins, which act by inducing tyrosine-specific protein phosphorylation (51-54). Consistent with these observations is the finding that both phorbol ester and various growth factors including bFGF, VEGF and PDGF stimulate the phosphorylation of cellular proteins on tyrosine residues whereas vanadate, perhaps by inhibiting tyrosine phosphatase(s), produces a marked increase in tyrosine phosphorylation.

Other derivatives of vanadate (bpV(phen); bpV(pic)) have been synthesized and have proven to be potent PTP inhibitors (1). These products were expected to promote endothelial cell proliferation, like vanadate. On the contrary, these agents have been shown to inhibit the proliferation of several cell types (4). In some cases, the cells are arrested at G2/M. The simplest hypothesis to explain this restriction is that PTPs controlling cell mitosis are targets for bpV(phen) and pbV (pic) inhibitors. The cdc25 protein was proposed as a candidate target for pV compounds (4).

International Patent Publication WO 95/19177 teaches the use of vanadate compounds for the treatment of proliferative disorders, metastasis and drug-resistant tumors. Although these vanadate compounds are stated to be anti-proliferative and anti-collagenolytic, no serious indication of any anti-angiogenic activity has been ascribed to them. This publication further shows that an anti-tumor effect is observed at dosages of vanadate higher than 5 mM. It is admitted that a concentration of vanadate compound of 1.3 mM or lower has no apparent anti-tumor effect.

Montesano et al. (48) teach, on the contrary, that vanadate compounds cause endothelial cells to proliferate. Hence, their findings would indicate that these compounds are pro-angiogenic and not anti-angiogenic.

US Patent 5,716,981 (Hunter et al.) mentions the use of vanadium compounds, namely oxovanadate, orthovanadate and vanadyl compounds, in anti-angiogenic applications. However, this reference provides no experimental evidence to substantiate the anti-angiogenic effects of these compounds. The compounds are stated to be possibly equivalent to Paclitaxel (an anti-angiogenic compound described in detail). Since the remaining body of the prior art suggests that vanadium compounds are pro-angiogenic, there is no enabling teaching in this patent to validate the use of these compounds as anti-angiogenics.

pV compounds are more powerful anti-tumor molecules than oxo compounds, such as vanadate. The direct antiproliferative activity of pV compounds on transformed cells is known (4). At concentrations found to be ineffective for vanadate compounds in International Patent Publication WO 95/19177, pV compounds are efficacious anti-tumor compounds.

Although the anti-tumor activity of vanadium compounds is known, their anti-angiogenic activity has not heretofore been disclosed in relation to either oxo or peroxo derivatives thereof.

### C) ENDOTHELINS:

Endothelins (ETs), a family of three isopeptides, acting as important regulators of the physiological state of mature blood vessels. They are the most potent vasoconstrictors identified to date. In addition, they are known to stimulate the proliferation of endothelial cells, smooth muscles, myocytes and fibroblasts, as well as the synthesis of various growth factors, including VEGF (55, 56).

ETs are also considered to be angiogenic factors involved in tumor development (57). Most tumor cells synthesize and secrete ETs (58-62). Patients affected by different cancers show elevated blood concentrations of ETs (63-65). A reduction in the expression of ET receptor type B (RET_{B}) decreases the growth of tumor cells incubated in the presence of ETs (65). As indicated above, ETs promote proliferation and migration of endothelial cells (66). Expression of ET ligands and of ET receptors (RET_{A} and RET_{B}) was observed in the endothelial cells of a plurality of tumors (67, 68). ETs act in an autocrine fashion, promoting local angiogenesis (69). ETs are further involved in hemodynamic changes that go along with metastatic development. For example, the ratio of arterial hepatic blood flow to portal vein blood flow is abnormally high in patients having hepatic metastasis from colorectal tumors (70). This high blood flow ratio is due to the presence of a humoral mediator as demonstrated *in vivo* (71).

A tumoral vascular bed has no innervation and consequently does not respond to vasoconstrictive drugs. However, these drugs decrease normal hepatic blood flow and increase blood flow in tumors. Since ETs are potent vasoconstrictive and angiogenic factors involved in vascular remodeling and tumor development, they may be responsible for these altered hemodynamics. An ET inhibitor may therefore be a valuable tool for controlling intratumor blood flow and for influencing the growth and degeneration of tumors. Additionally, such an inhibitor could be useful in verifying whether the anti-tumor effect of vanadium compounds involves ETs.

### D) ANGIOPLASTY:

ETs are also known to stimulate the production of extracellular matrices by endothelial cells (72, 73). That is frequently observed upon vascular reconstruction or angioplasty (74, 75). This effect is particularly deleterious following vascular trauma due to restenosis. In 30-50% of patients, restenosis is characterized by the reexpansion of atherosclerotic lesions. The causes of this vascular disorder are due to a local vascular blockage caused by cell proliferation, cell migration and extracellular matrix production. Recently, ETs have been shown to be major players in vascular remodelling, particularly in such conditions as long term atherosclerosis, cardiac hypertrophy (congestive heart failure), hypertension (pulmonary and other), renal problems and certain systemic dysfunctions (76-80). ETs are also strongly involved in coronary and brain vasospasm leading to ischemia and a reduced rate of survival (81, 82). Thus, because of the potent vasoconstrictor and nitogenic activities of ETs, inhibitors of ET production would be expected to be generally useful as anti-hypertensive and anti-proliferative agents, and particularly useful prior to, during and after vascular surgery.

### SUMMARY OF THE INVENTION

The present invention provides pV compounds that are useful against angiogenesis, restenosis and endothelin production. These molecules comprise vanadium, and one peroxo groups. Molecules comprising peroxo groups are the more potent anti-angiogenic molecules. Preferably, the molecules also contain an ancillary ligand, which includes any molecule capable of binding the transition metal atom (usually, through bonds involving oxygen and nitrogen). Phenanthroline, picolinic acid, bipyridine, oxalic acid, 4,7-dimethyl-phenanthroline and peptides are examples of such ligands.

All these molecules can be used to inhibit the formation of new blood vessels and/or control systemic and local levels of endothelins (ETs) in the reparation of existing blood vessels.

The molecules containing peroxo anions are more powerful than their oxo counterparts. Therefore, the former can be used at much lower concentrations to reduce the toxicity that results from overexposure to transition metals (4).

Oxo transition metal complexes include oxo complexes such as vanadate, tungstate, molybdate, and vanadyl complexes, such as the following: methavanadate (VO₃⁻), orthovanadate (VO₄³⁻), salts thereof, vanadyl compounds (VO²⁺) like vanadyl acetyl acetonate and vanadyl sulfate. Similar complexes exist for other transition metals. Other suitable tungsten and molybdenum complexes include hydroxo derivatives derived from glycerol, tartaric acid and sugars, for example. The peroxo transition metal complexes include any oxidizing agent capable of combining with the transition metal. As such, the preferred peroxides comprise the following: t-butylhydroperoxide, benzoyl peroxide, m-chloroperoxibenzoic acid, cumene hydroperoxide, peracetic acid, hydroperoxiloneic acid, ethyl peroxide, pyridine peroxide and hydrogen peroxide.

The general structure of the compounds of the present invention is the following: 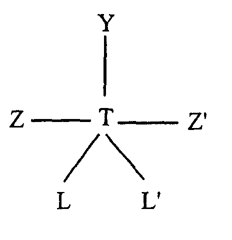 wherein:
T is vanadium;
Y is oxygen or hydroxyl;
Z and Z' are independently selected from oxygen and peroxide; and
L and L' are any group which can donate an electron pair.

In a preferred embodiment, Y is oxygen, Z and Z' are peroxide and L and L' are the nitrogen atoms of 1,10-phenanthroline.

In another preferred embodiment, Y is oxygen, Z and Z' are peroxide and L and L' are nitrogen or oxygen atoms of picolinic acid.

In yet another preferred embodiment, Y is oxygen, Z and Z' are peroxide and L and L' are nitrogen atoms of 2,2'-bipyridine.

The above pV compounds are potent anti-angiogenics, since they inhibit endothelial cell proliferation. They further inhibit neovascularization and the production of endothelins.

Moreover, administration of the pV compound bpV(bipy) in the rat model revealed a significant reduction in the degree of post-angioplastic vascular remodeling of the carotid artery. Additionally, the pV compound bpV(phen) was found to be a potent immunomodulator based on its capacity to induce cytokine and chemokine gene expression, to enhance cellular recruitment in response to an agonist and consequently act as an adjuvant in the context of vaccination.

Other objects, advantages and features of the present invention will become apparent upon reading the following non-restrictive description of preferred embodiments thereof, with references to the accompanying drawings.

### DESCRIPTION OF THE PREFERRED EMBODIMENTS OF THE PRESENT INVENTION

This invention will now be described by referring to specific embodiments and the appended Figures, which purpose is to illustrate the invention rather than to limit its scope.

### BRIEF DESCRIPTION OF THE FIGURES

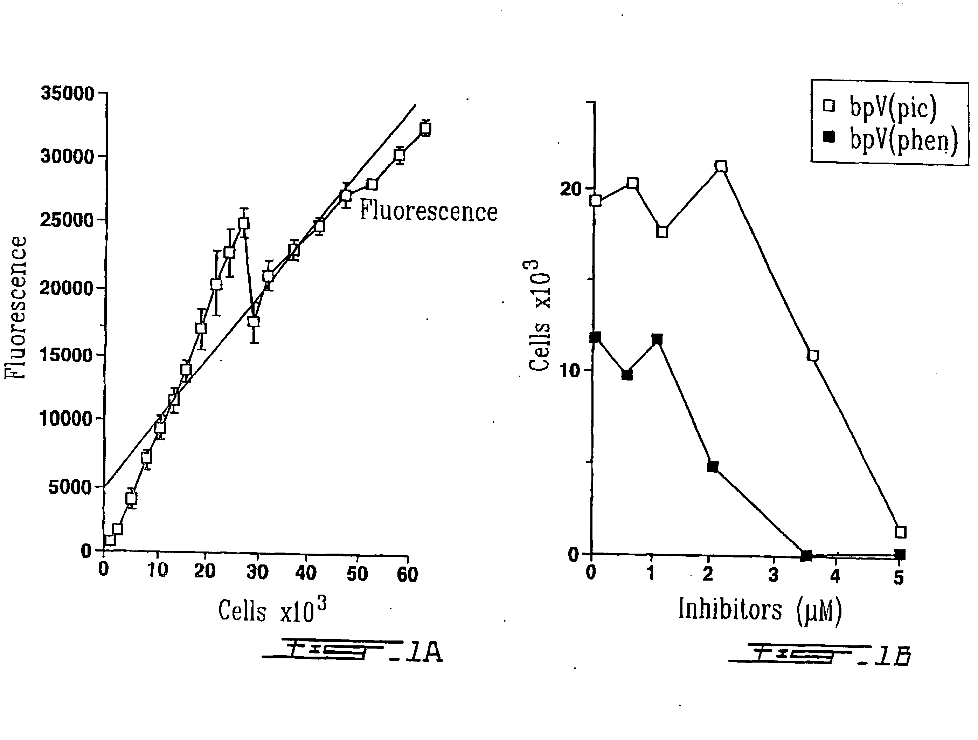

**Figure 1:** illustrates the concentration-response effect of bpV(phen), bpV(pic) and vanadate on the growth rate of human vein umbilical endothelial cells (HUVECs).

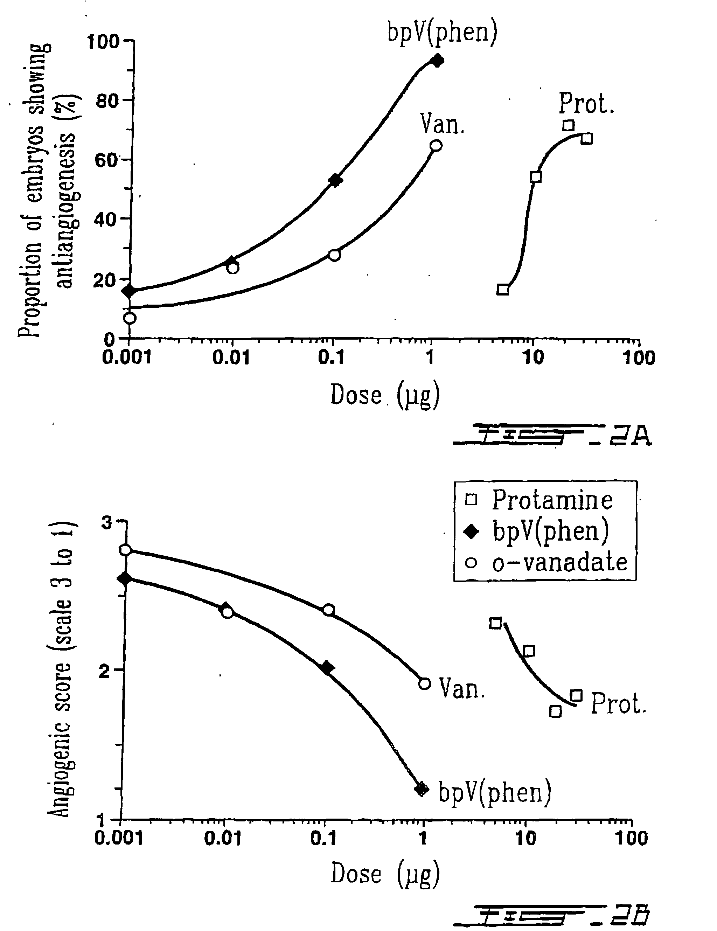

**Figures 2:** A and B show an anti-angiogenic response to bpV(phen), orthovanadate (Van) and protamine (Prot) using a vascularization test on the vitelline membrane of chick embryos. Each point represents a minimum of 15 embryos (15 to 28). In Figure 2A), neovascularization was assessed using N/+ scoring system; the proportion of embryos showing anti-angiogenesis increased with dosage, bpV(phen) being the most potent agent. In Figure 2B, neovascularization was assessed using the 1-2-3 scoring system; the angiogenic score decreased with dosage, bpV(phen) being the most potent agent.

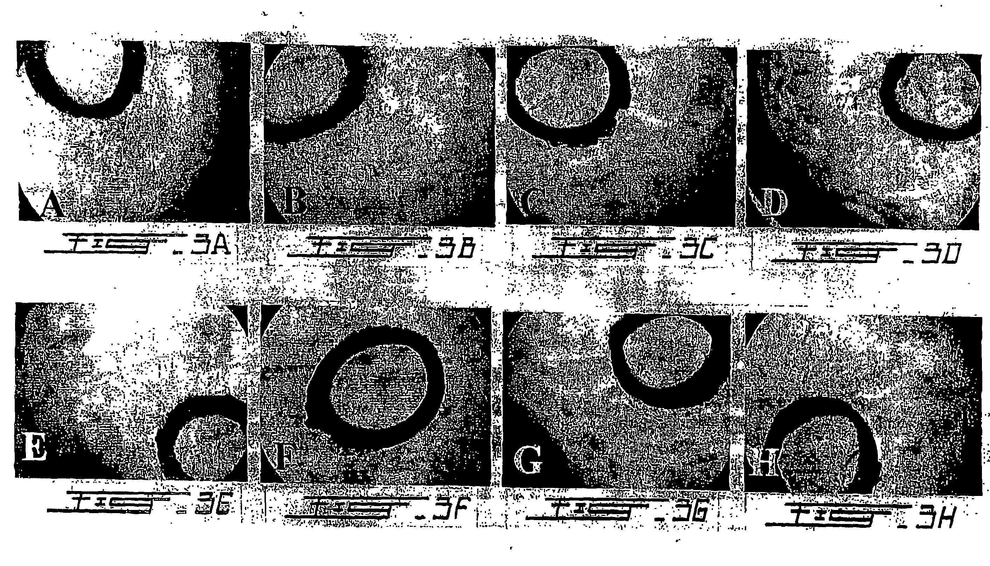

**Figure 3:** Micrographs of the cell migration from aortic rings embedded in fibrin (x12). Compared to the sample control (A), cell migration was impaired in the presence of doses of bpV(phen) (1, 3.5 µM; B, C) with a strong inhibition at 3.5 µM (C). In the presence of bpV(pic) (3.5, 10, 25 µM; D, E, F), inhibition was seen at 10-25µM. In the presence of orthovanadate, partial inhibition was reached at 25 µM (G) with no effect at 3.5 µM (H). The migration of well-defined microvessels in the presence of bpV(pic) is evident at 10 µM, while in the control sample very thin cell strands were frequently observed.

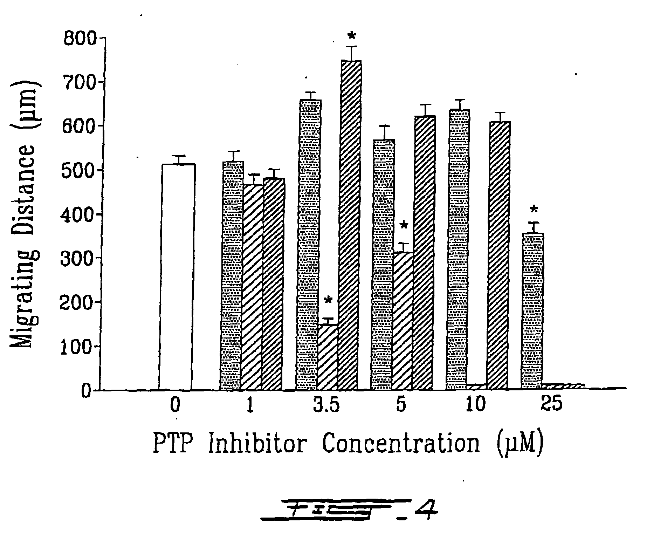

**Figure 4:** Quantification of the cell migration from aortic rings in the presence of orthovanadate (plain bars); bpV(phen) (light gray bars); and bpV(pic) (dark gray bars). The control appears as an empty bar. Means and standard errors of the means are presented. The Student-Newman-Keuls method was used for statistical analysis (p value=0.001)

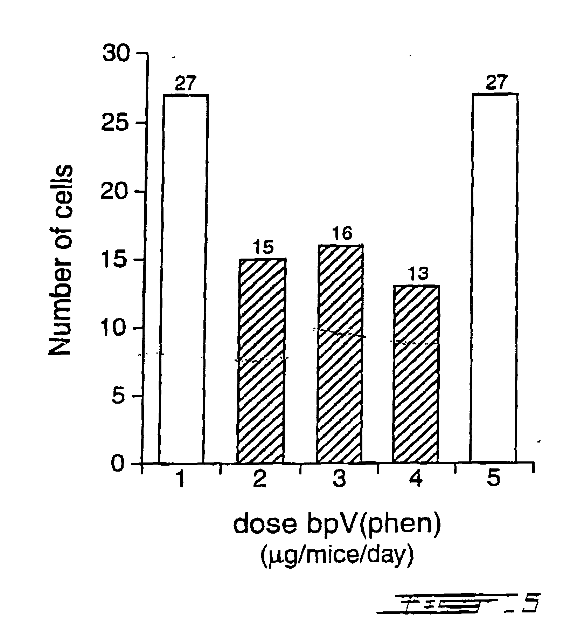

**Figure 5:** bpV(phen) inhibits angiogenesis *in vivo* in the s.c. Matrigel assay. Graph showing the results of an experiment expressed as the number of cells that migrated in three microscopic fields for each s.c. Matrigel plug. Fields were equidistant from the edge of the plug. (1) Four mice that did not receive any treatment after the Matrigel plug implantation. (2) Four mice that received daily administration of 10 µg bpV(phen) during the 7 days post-implantation. (3) Four mice that received daily administration of 50 µg bpV(phen) during the 7 days post-implantation. (4) Four mice that received daily administration of 100 µg bpV(phen) during the 7 days post-implantation. (5) Four mice that received daily administration of PBS during the 7 days post-implantation.

### A) PEROXOVANADIUM COMPOUNDS and ANGIOGENESIS

### 1- Peroxovanadium compounds inhibit the proliferation of endothelial cells and the formation of tubular structures

Human umbilical vein endothelial cells (HUVECs) were extracted with collagenase-controlled digestion, as previously described (105). Pure HUVECs were used before the fourth passage (trypsin-EDTA at each passage). The cells were analyzed for their capacity to incorporate di-acetyl LDL and to be labelled with factor VIII-related antigen.

Endothelial cells were plated at a density of 2500 cells/cm² in a sterile plate coated with gelatin. Cells were cultured in complete medium (M199: heparin (90 µg/ml) or L-glutamine (2mM), bicarbonate, FBS (20%) and ECGS (100 µg/ml)) for 24 hours to ensure cell adhesion. Then, cells were washed 3 times with PBS and culture medium was added according to experimental conditions. The last PBS wash was considered as time t=0.

Cell proliferation was evaluated with the amount of DNA present in the petri dishes. Each experiment was performed in triplicate. The culture medium was changed daily. After 96 hours in culture, cells were lysed with Na-Citrate-SDS solution and incubated with Hoescht 33358. Samples were read at 365 nm with a spectrofluorometer.

The results show a dose-response inhibition of endothelial cell proliferation with the pV compounds (Figure 1). The approximate IC₅₀ is 2 µM for bpV(phen) and 3.5 µM for bpV(pic).

Alternatively, HUVECs cultured in a fibrin matrix can form 3-dimensional tubular-like structures in the presence of serum (106). This assay was performed in the presence of either bpV(phen), bpV(pic) or vanadate to assess the influence of each one of these compounds on HUVEC differentiation and organization (Table 1). HUVECs were seeded on the bottom of gelatin-coated wells at high density to provide a confluent monolayer at 48 hours.

Then, 50 000 HUVECs/ml were embedded in fibrinogen solution prior to polymerization. The fibrin matrix was covered with culture medium containing the molecule to be tested, while culture medium without any of these molecules served as controls. Cell behavior was observed periodically by phase contrast microscopy. After 21 days in culture in the presence of 1 µM bpV(phen), cord-like structures (or cords), tubelike structures (or tubes) and stellate structures (or Stell.struct.) were observed. At higher doses (2 and 3.5 µM), fragmented cord-like structures were apparent. In the presence of bpV(pic), cords and tubes were observed at 1, 2 and 3.5 µM. In the presence of orthovanadate, cords were still apparent at 10 µM, and at 25 µM dead cells were sparsely distributed. These results suggest that pV compounds interfere with endothelial cell organization and terminal differentiation. Furthermore, the nature of the ancillary ligand is important since bpV(phen) is more potent in inhibiting tube formation than bpV(pic).

The results show that vanadate has an anti-angiogenic effect inasmuch as the stellate structures are affected (organization and terminal differentiation). Vanadate is at least 3 and 5 times less potent than bpV(pic) and bpV(phen), respectively. The anti-angiogenic effect observed with vanadate is in contradiction to the teachings of Montesano (48).

Indeed, it was observed that at low doses, all the tested vanadium compounds seemed to be pro-angiogenic, while at higher doses (i.e., at almost double the dose), the same compounds were clearly anti-angiogenic. The type of radical L, L' greatly affects the potency of the vanadium compounds; for example, phenanthroline was found to be twice as potent as picolinic acid.

**TABLE I:**

| **Effects of bpV(phen), bpV(pic) and Vanadate on HUVEC** **Differentiation and Organization** | | | | |
|---|---|---|---|---|
| | Doses (µM) | cords | tubes | Stell. struct. |
| bpV (phen) | 0 | +++ | +++ | +++ |
| | 1 | +++ | +++ | +++ |
| | 2 | ++ | + | - |
| | 3.5 | - | - | - |
| bpV (pic) | 0 | +++ | +++ | +++ |
| | 1 | +++ | +++ | +++ |
| | 2 | +++ | +++ | +++ |
| | 3.5 | ++ | + | - |
| van | 0 | +++ | +++ | +++ |
| | 2.5 | +++ | +++ | +++ |
| | 10 | +++ | +++ | - |
| | 25 | † | † | † |

| | | | | |
|---|---|---|---|---|
| † Dead cells | | | | |

### 2- Rat aortic ring assay

Rat aorta rings were embedded in fibrin matrix as previously described (107). Thoracic aortas excised from adult rats were rinsed in Hank's balanced salt solution (HBSS), and cleansed of periadventitial. One-mm long aortic rings were sectioned and rinsed in culture medium. Each of the aortic rings were then embedded in fibrin gel matrix. Migration of microvessels in fibrin gel was quantified by measuring the distance from the external surface of rat aortic rings towards the migrating vessels. Measurements were performed at day 15 by digitizing morphometry with a NIH image analysis system (Fig. 3 and 4).

### 3- Peroxovanadium compounds inhibit neovascularization ex ovo

### a. Definition of the test-system:

The normal development of a chick embryo involves the formation of an external vascular system which is located in the vitelline membrane and which carries nutrients from the vitellus (yolk of the egg) to the developing embryo. When placed onto the vitelline membrane, anti-angiogenic substances can inhibit blood vessel development in the vitelline membrane. To facilitate access to the vitelline membrane, chick embryos were transferred to a sterile culture box (Petri dish) and placed in a humidity- and temperature-controlled incubator. Embryos could then develop in this *ex ovo* condition for several days.

An aliquot of the tested compound was mixed with a methylcellulose solution and allowed to air-dry into thin discs. Methylcellulose forms an inert matrix from which the tested compound can diffuse slowly. Methylcellulose discs containing the tested compound were placed on the external border of the vascular perimeter of the vitelline membrane where the angiogenic process was still active.

The effects of the discs containing the tested compound on proximal vascular development were, in all cases, compared with those of discs containing control buffer. The discs were placed on the embryos' vitelline membrane on Day 0 or Day 1 of the *ex ovo* growth process; at this point, only beginnings of the main blood vessels are invading the vitellus. The embryos were then put in culture conditions until vascularization was assessed (approximately 24 h). Control buffer- and compound-containing discs were in all cases added simultaneously on the vitelline membrane of the same embryo. Both discs were arranged in a symmetrical fashion with respect to the cephalo-caudal axis of the embryo in order to minimize inter-individual variations when comparing the tested compounds with controls.

### b. Anti-angiogenic activity:

Embryonic vascularization tests (EVTs) were performed using different concentrations of protamine (5 to 20 µg) as a positive control or a tested compound (0.001 to 10 µg). After one day in culture, the level of vascularization in the area covered by the discs was graded by a pair of scientists in the usual blind fashion. To facilitate the location of the discs, black O-rings were placed around them just after their placement on the vitelline membrane. The evaluation scale for the EVTs was based on two different scoring systems.

### Assessment of blood vessel formation:

Blood vessel formation was assessed in a blind fashion. The areas of the vitelline membranes that lay beneath the methylcellulose discs were scored for the degree of vascularization, using two scoring systems (" N/+" or " 1-2-3"). The following selection criteria were used:

### N/+ system:

A score of "N" for "Normal" was attributed when all the following conditions were met:

Blood vessels in the evaluated area grew along their path with no abnormal deviation. Collateral branching density was normal and the growth path of the lateral branches was also normal.

A score of " +" was attributed when at least one of the following conditions was met:
- Major blood vessels grew across the evaluated area but their paths were clearly affected (winding).
- Major blood vessels grew across the evaluated area but collateral branching density was clearly diminished.
- Major blood vessels penetrated the evaluated area but their growth path rapidly deviated.
- A kink was observed in the blood vessel.
- Major blood vessels penetrated the evaluated area but were stunted. No growth was observed beyond that point.
- A drastic deviation in the growth path occurred when major blood vessels reached the proximal ridge of the disc.

### 1-2-3 grading scale system:

A score of 3 was attributed when the following conditions were met:
- Blood vessels, present in the evaluated area, grew along their paths with no abnormal deviation. Collateral branching density was normal and the growth paths of the lateral branches were also normal.

A score of 2 was attributed when at least one of the following conditions was met:
- Major blood vessels grew across the evaluated area but their paths were clearly affected (winding).
- Major blood vessels grew across the evaluated area but collateral branching density was clearly diminished.

A score of 1 was attributed when at least one of the following conditions was met:
- Major blood vessels penetrated the evaluated area but their growth paths were rapidly deviated.
- A kink was observed in the blood vessels.
- Major blood vessels penetrated the evaluated area but were stunted. No growth was observed beyond that point.
- A drastic deviation in the growth paths occurred when major blood vessels reached the proximal ridge of the disc.

A score of 3 meant that blood vessel development was normal whereas a score of 1 indicated the greatest degree of angiostatic activity.

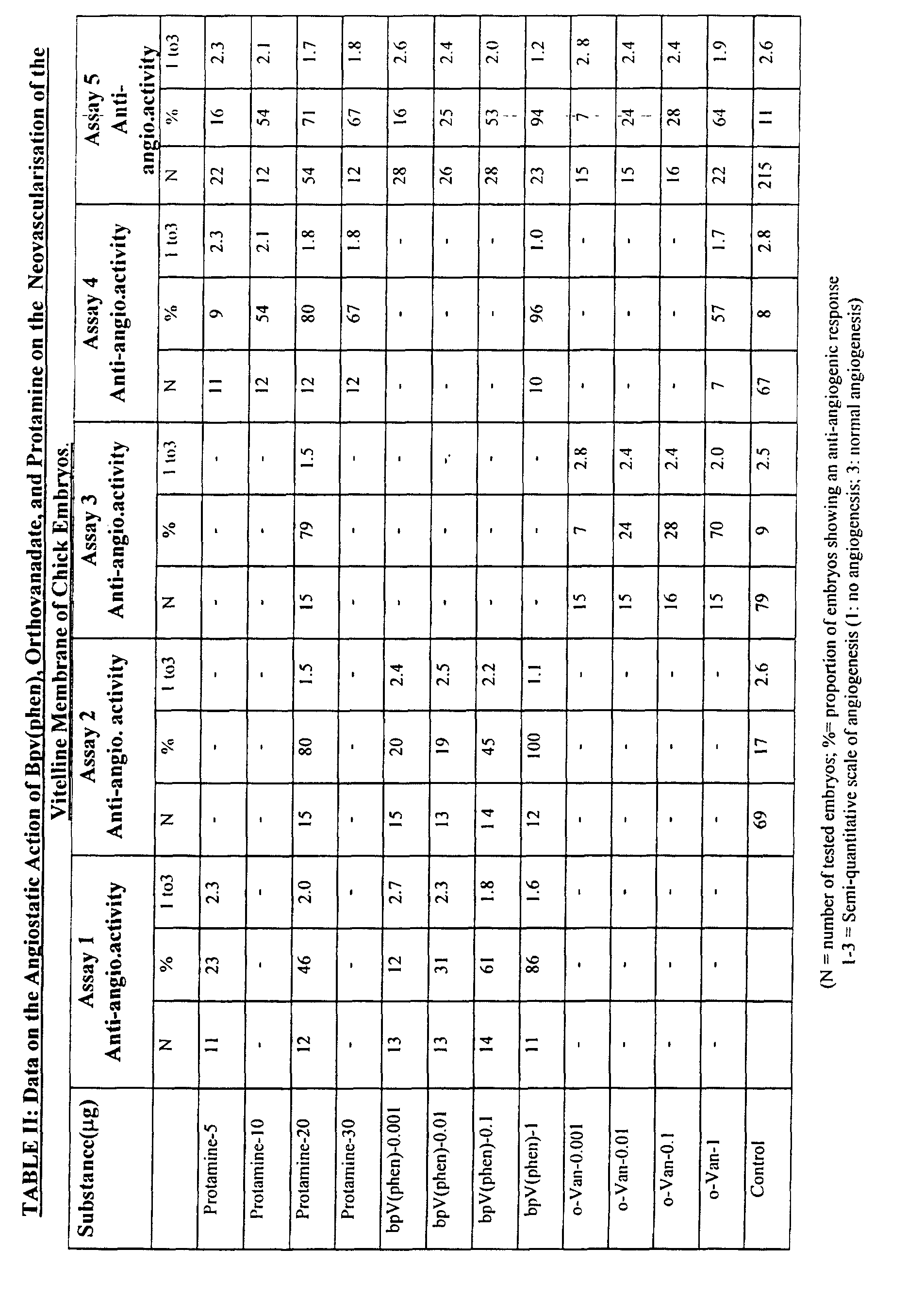

A dose-response inhibition was obtained with protamine, bpV(phen), and orthovanadate (Figure 2). The approximate IC₅₀ is 0.1 µg for bpV(phen), 0.6 µg for orthovanadate, and 11 µg for protamine (regardless of the scoring system). A summary of the experimental data collected is shown in Table II.

The results show that bpV(phen) is a potent inhibitor of the angiogenic process. Prior art on the subject (vanadate) taught the opposite, namely that vanadium compounds promote angiogenesis.

### 4- In vivo Matrigel plug assay

This assay was performed as previously described (108). Briefly, Matrigel (liquid at 4°C) was mixed with 200 ng/ml ECGS and injected s.c. into four C57B1/6N female mice (1 ml/mice). After injection, the Matrigel polymerized to form a plug. After 7 days, the animals were sacrified, and the Matrigel plugs were removed and fixed in 10% neutral-buffered formalin solution (Sigma Chemical Co.) and embedded in paraffin. Histological sections were stained with Masson's trichrome and angiogenesis was scored by counting the number of cells that migrated in three microscopic fields for each s.c. Matrigel plug.

Experiments were performed to determine whether bpV(phen) inhibits vessel formation *in vivo*. Endothelial cells invade and form vessels in Matrigel plugs containing angiogenic factors ECGS (109). Animals were divided into five groups (4 mice/group): two control groups that received no treatment or daily i.p. PBS injection, respectively, and three groups that received daily administration of various doses (10, 50 and 100 µg) of bpV(phen), from day 1 to day 7 (intraperitoneal daily administration of 100 µl solution). The results show that bpV(phen) is a potent angiogenesis inhibitor *in vivo* (Fig. 5). Daily administration of 10 µg/mice or higher doses decreased by half the number of endothelial cells having invaded Matrigel plugs.

The angiogenic potential of pV compounds was tested using *in vitro, ex ovo*, and *in vitro* systems of analysis. The results show that in contrast to what might have been expected from the teaching of the prior art, pV compounds are potent inhibitors of the angiogenic process (see below), and this includes high doses of vanadate.

### B) PEROXOVANADIUM COMPOUNDS and THE PRODUCTION OF ENDOTHELINS

The data presented in Table III show the effect of bpV(phen) on the plasmatic levels of ETs. In this study, rats were injected intraperitoneally with bpV(phen) (0.5 mg/100g b.w.) 16 hours before the administration of either insulin or vehicle. Two minutes following insulin administration (1.5 µg/100g b.w, or vehicle), the plasma levels of ETs were determined. Insulin administration induced a strong increase of seric ET concentration (55). This increase was completely abolished by bpV(phen). In addition, bpV(phen) decreased the insulin-stimulated levels of plasmatic ETs below control levels. These results suggest that bpV(phen) inhibits the insulin-induced release of ETs that subsequently can lead to diabetic complications like vasculopathy and nephropathy.

**TABLE III:**

| **Effect of bpV (phen) on Endothelin Plasma Levels** | |
|---|---|
| **Plasma endothelins (pg/ml)** | |
| Control | 113.41 ±10.91 |
| Insulin | 253.10 |
| Insulin, bpV(phen) | 77.68 ±3.08 |

ETs (ET-1, ET-2, ET-3) were measured by RIA (Amersham kit RPA 555) after lyophilization and extraction on C2 columns (500 mg). Results are mean ±SEM, p<0.0209, control (n=4) vs Insulin, pV (n=3).

### C) PEROXOVANADIUM CONPOUNDS and RESTENOSIS

The data presented in Table IV show the effect of the pV compound bpV(bipy) on restenosis. Male Sprague-Dawley rats (325-350g) were anaesthetized with Rogarsetic, a mixture of ketamine hydrochloride and xylazine hydrochloride. The animals were divided into two groups of 14-31 rats that underwent balloon angioplasty of the left carotid artery, with or without treatment (109). The treated group received bpV (bipy) (10 mg / kg / i.p. b.i.d.) for 14 days, starting on the day of the angioplasty. Every animal in each group underwent balloon angioplasty of the left external carotid whereas the contra-lateral carotid served as a control for each animal. The left carotid artery balloon angioplasty was performed under aseptic conditions. The left external carotid was isolated and an arteriotomy was performed. A 2F Fogarty arterial embolectomy catheter was then inserted in the left common carotid and positioned near the aortic arch. The balloon was then inflated and retracted with a twisting motion to its insertion point. This procedure was repeated twice and the external carotid was ligated. There were no differences in the lumina (L), intima (I), media (M), the ratio of I/M and calculated value of (M - L / P) as a measure of neointimal proliferation (NIP) between normal, non-ballooned carotid artery of treated and non-treated rats, serving as controls (data not shown). Treatment caused a 32% decreased in NIP and the I/M ratio, a 21 % reduction in the thickness of the I and a 28% increase in the opening of the L. Thus, treatment with bpV (bipy) revealed a significant reduction in the degree of post-angioplastic vascular remodeling of the carotid artery in the rat model.

The above results illustrate the antiangiogenic and anti-restenosis potential of vanadium compounds. The administration of pV compounds could be used to control the progression of several angiogeno-dependent conditions as well as post-angioplastic vascular remodeling. Since vanadates are anti-tumor compounds, the present pV compounds should function as anti-tumor therapeutic agents, and part of this activity should be due to an anti-angiogenic effect. Pharmaceutical compositions would comprise potent pV compounds capable of achieving an extracellular concentration of about 0.1 to 100 µM, preferably 2-40 µM. In rats, a dose of 20 µmoles per kilogram was successful in reversing the endothelin increase after insulin administration. Moreover, pV compounds, which as previously stated are more potent and provoke less side effects than the vanadium "oxo" compounds, can be administered to achieve the desired dosage. For example, see *in vivo* effects on blood glucose levels have been reported (1, 3).

**TABLE IV:**

| **Effect of bpV (bipy) on Restenosis** | | | | | |
|---|---|---|---|---|---|
| | NIP mm | I mm² | M | I / M | L mm² |
| Control | 0.064 | 0.138 | 0.090 | 1.546 | 0.090 |
| bpV(bipy) | 0.045 | 0.112 | 0.104 | 1.086 | 0.112 |
| p < x | 0.002 | 0.04 | 0.005 | 0.003 | 0.003 |
| Abbreviations: I, intima; M, media; NIP, neointimal proliferation; L, lumina. | | | | | |

### LIST OF REFERENCES

1. Posner, B.I., Faure, R., Burgess, J.W., Bevan, A.P., Lachance, D., Zhan-Sun, G., Fantus, I.G., Ng, J.B., Hall, D.A., Lum, B.S. & Shaver, A. (1994). Peroxovanadium compounds: a new class of potent phosphotyrosine-phosphatase inhibitors which are insulin mimetics. J. Biol. Chem. 269: 4596-4604.
2. Shaver, A., Ng, J.B., Hall, D.A., & Posner, B.I. (1995). The chemistry of peroxovanadium compounds relevant to insulin mimesis. Mol. Cell. Biochem. 153: 5-15.
3. Bevan, A. P., Burgess, J. W., Yale, J. F., Drake, P. G., Lachance, D., Baquiran, G., Shaver, A., & Posner, B. I. (1995). In vivo insulin mimetic effects of pV compounds: role for tissue targeting in determining potency. Am J Physiol 268: E60-66..
4. Faure, R., Vincent, M., Dufour, M., Shaver, A. & Posner, B. (1995). Arrest at the G2/M transition of the cell-cycle by protein-tyrosine phosphatase inhibition: Studies on a neuronal and a glial cell line. J. Cell. Biochem. 58: 389-401.
5. Olivier, M, Romero-Gallo B. J., St-Laurent, R., Racette, N., Stevenson, M., Jacobs, P., Posner, B.I., Tremblay, M., & Faure, R. (1998). Modulation of INFg induced macrophage activation by phosphotyrosine phosphatase inhibition. J. Biol. Chem. 273: 13944-13949.
6. Reydi, M. A. & Schwartz, S. M. (1981) Endothelial regeneration: Time course of initial changes after small defined injury to rat aortic endothelium. Lab. Invest. 44: 301-308.
7. Schwartz, S. M. & Liaw, L. (1993) Growth control and morphogenesis in the development of pathology of arteries. J. Cardiovasc. Pharmacol. 21 (suppl): S31-S49.
8. Auerback, R. (1994). Angiogenesis inhibition: a review. Pharmac. Ther. 63: 265-311.
9. Folkman, J. (1995). Angiogenesis in cancer, vascular, rhumatoid and other diseases. Nature Med. 1: 27-31.
10. Schreiber, A.B., Winkler, M.E. & Derynck, R. (1986). Transforming growth factor-alpha: a more potent angiogenic factor than epidermal growth factor. Science 232: 1250-1254.
11. Roberts, A.B., Sporn, M.B., Assoian, R.K., Smith, J.M., Roche, N.J. & Wakefield, L.M. (1986). Transforming growth factor type beta: rapid induction of fibrosis and angiogenesis in vivo and stimulation of collagen formation in vitro. Proc. Natl. Acad. Sci. USA 84: 4167-4171.
12. Fràter-Schröder, M., Risau, W., Hallmann, R., Gautschi, R. & Bohlen, P. (1987). Tumor necrosis factor type-alpha, a potent inhibitor of endothelial cell growth in vitro, is angiogenic in vivo. Proc. Natl. Acad. Sci. USA 84: 5277-5282.
13. Fett, J.W., Strydom, D.J., Lobb, R.R., Alderman, R.M., Riordn, J.F. & Vallee, B.L. (1985). Isolation and characterization of angiogenin, an angiogenic protein from human carcinoma cells. Biochemistry 24: 5480-5488.
14. Ziche, M., Jones, J. & Gullino, P. (1982). Role of prostaglandin E1 and copper in angiogenesis. J. Natl. Cancer Inst. 69: 475-482.
15. Dobson, D.E., Kambe, A., Block, E., Dion, T., Lu, H. & Castellot, Jr. J.J., Speigelman, B.M. (1990). 1-Butyrylglycerol: a novel angiogenesis factor secreted by differentiating adipocytes. Cell 61: 223-230.
16. Baird, A. & Durkin, T. (1986). Inhibition of endothelial cell proliferation by type-beta transforming growth factor: interaction with acidic and basic fibroblast growth factors. Biochem. Biophys. Res. Commun. 138: 476-482.
17. Fràter-Schröder, M., Muller, G., Birchmeier, W. & Bohlen, P. (1986). Transforming growth factor-beta inhibits endothelial cell proliferation. Biochem. Biophys. Res. Commun. 137: 295-302.
18. Folkman, J., & Klagsbrun, M. (1987). Angiogenic factors. Science 235: 442-447.
19. Klagsbrun, M. & D'Amore, P.A. (1991). Regulators of angiogenesis. Annu. Rev. Physiol. 53: 217-232.
20. Gospodarowicz, D., Ferrara, N., Schweigerer, L. & Neufeld, G. (1987). Structural characterization and biological functions of fibroblast growth factor. Endocr. Rev. 8: 95-104.
21. Thomas, K., Rios Candelore, M., Gimenez-Gallego, G., Di Salvo, J., Bennet, C., Rodkey, J. & Fizpatrick, S. (1985). Pure brain-derived acidic fibroblast growth factor is a potent vascular endothelial cell mitogen with sequence homology to interleukin-1. Proc. Natl. Acad. Sci. USA 82: 6409-6416.
22. Miyazono, K., Okabe, T., Urabe, A., Takata, F. & Heldin, C.-H. (1987). Purification and properties of an endothelial cell mitogen from human platelets. J. Biol. Chem. 262: 4098-4104.
23. Ishikawa, F., Miyazono, K., Hellman, U., Drexler, H., Westemdt, C., Hagiwara, K., Usuki, K., Takaku, F., Risau, W. & Heldin, C.-H. (1989). Identification of angiogenic activity and the cloning and expression of platelet-derived endothelial cell growth factor. Nature 338: 557-561.
24. Leung, D.W., Cachianes, G., Kuang, W.-J., Goeddel, D.V. & Ferrara, N. (1989). Vascular endothelial growth factor is a secreted angiogenic mitogen. Science 246: 1306-1312.
25. Houck, K.A., Ferrara, N., Winer, J., Cachianes, G., Li, B. & Leung, D.W. (1991). The vascular endothelial growth factor family: Identification of a fourth molecular species and characterization of alternative splicing of RNA. Mol. Endocrinol. 5: 1806-1814.
26. Breier, G., Albrecht, U., Sterrer, S. & Risau, W. (1992). Expression of vascular endothelial growth factor during embryonic angiogenesis and endothelial cell differentiation. Development 114: 521-532.
27. Bussolino, F., Albini, A., Garnussi, G., Presta, M., Viglietto, G., Ziche, M. & Persico, G. (1996). Role of soluble mediators in angiogenesis. Eur. J. Cancer 32A: 2401-2412.
28. Colville-Nash, P.R. & Willoughby, D.A. (1997). Growth factors in angiogenesis: current interest and therapeutic potential. Mol. Med. Today. 3: 14-23.
29. Knighton, D.R., Hunt, T.K., Scheuenstuhl, H., Halliday, B.J., Werb, Z. & Banda, M.J. (1983). Oxygen tension regulates the expression of angiogenesis factor by macrophages. Science 221: 1283-1285.
30. Ladoux, A. & Frelin, C. (1993). Hypoxia is a strong inducer of vascular endothelial growth factor mRNA expression in the heart. Biochem. Biophys. Res. Commun. 195: 1005-1010.
31. Nomura, M., Yamagishi, S.I., Harada, S.I., Hayashi Y., Yarnashima, T., Yamashita, J. & Yamamoto H. (1995). Possible participation of autocrine and paracrine vascular endothelial growth factors in hypoxia-induced proliferation of endothelial cells and pericytes. J. Biol. Chem. 270: 28316-28324.
32. Walgenbach, K.J., Gratas, C., Shestak, K.C. & Becker, D. (1995). Ischaemia-induced expression of bFGF in normal skeletal muscle: a potential paracrine mechanism for mediating angiogenesis in ischaemic skeletal muscle. Nature Med. 1: 453-459.
33. Lew, A.P., Lew, N.S., Iliopoulos, O., Jiang, C., Kaeling Jr., W.G. & Goldberg, M.A. (1997). Regulation of vascular endothelial growth factor by hypoxia and its modulation by the von Hippel-Lindau tumor suppressor gene. Kidney Int. 51: 575-578.
34. Sandner, P., Wolf, K., Bergmaier, U., Gess, B. & Kurtz, A. (1997). Induction of VEGF and VEGF receptor gene expression by hypoxia: Divergent regulation in vivo and in vitro. Kidney Int. 51: 448-453.
35. Ferrara, N. (1995). Missing link in angiogenesis. Nature 376: 467.
36. Koch, A.E., Halloran, M.M., Haskell, C.J., Shaw, M.R. & Polverini, P.J. (1995). Angiogenesis mediated by soluble forms of E-selectin and vascular cell adhesion molecule-1. Nature 376: 517-519.
37. Polverini, J.P., Cotran, R.S., Gimbrone Jr, M.A. & Unanue, E.R. (1977). Activated macrophages induce vascular proliferation. Nature 269: 804-806.
38. Sunderkotter, C., Steinbrink, K., Goebeler, M., Bhardwaj, R. & Sorg, C. (1994). Macrophages and angiogenesis. J. Leukocyte Biol. 55: 410-422.
39. Polverini, P.J. (1996). How the extracellular matrix & macrophages contribute to angiogenesis-dependant diseases. Eur. J. Cancer 14: 2430-2437.
40. Gross, J.L., Moscatelli, D., Jaffe, E.A. & Rifkin, D.B. (1982). Plasminogen activator and collagenase production by cultured capillary endothelial cells. J. Cell Biol. 95: 974-981.
41. Moscatelli, D.A., Rifkin, D.B. & Jaffe, E.A. (1985). Production of latent collagenase by human umbilical vein endothelial cells in response to angiogenic preparations. Exp. Cell Res. 156: 379-390.
42. Ingber, D.E. (1991). Extracellular matrix and cell shape: potential control points for inhibition of angiogenesis. J. Cell. Biochem. 47: 236-241.
43. Ingber, D.E. (1992). Extracellular matrix as a solid-state regulator in angiogenesis: identification of new targets for anti-cancer therapy. Sem. Cancer Biol. 3: 57-63.
44. Bischoff, J. (1995). Approaches to studying cell adhesion molecules in angiogenesis. Trends Cell Biol. 5: 69-74.
45. Eisenstein, R. (1991). Angiogenesis in arteries: review. Pharmac. Ther. 49: 1-19.
46. Folkman, J. & Ingber, D. (1992). Inhibition of angiogenesis. Cancer Biol. 3: 89-96.
47. Bicknell, R. (1994). Vascular targeting and the inhibition of angiogenesis. Ann. Oncol. 5: S45-S50.
48. Montesano, R., Pepper, M.S., Belin, D., Vassalli, J.-D. & Orci, L. (1988). Induction of angiogenesis in vitro by vanadate, an inhibitor of phosphotyrosine phosphatases. J. Cell. Physiol. 134: 460-466.
49. Montesano, R. & Orci, L. (1985). Tumor-promoting phorbol esters induce angiogenesis in vitro. Cell 42: 469-477.
50. Montesano, R., Vassalli, J.D., Baird, A., Guillemin, R. & Orci, L. (1986). Basic fibroblast growth factor induces angiogenesis in vitro. Proc. Natl. Acad. Sci. USA. 83: 7297-7301.
51. Sefton, B.M. & Hunter, T. (1984). Tyrosine protein kinases. Adv. Cyclic Nucleotide Protein Phosphorylation Res. 18: 195-203.
52. Hunter, T. & Cooper, J.A. (1985). Protein-tyrosine kinases. Annu. Rev. Biochem. 54: 897-930.
53. Hunter, T. & Cooper, J.A. (1986). Viral oncogenes and tyrosine phosphorylation. In: The Enzymes, Vol. 17. P.D. Boyer and E.G. Krebs, eds. Academic Press, Orlando, Florida, pp. 191 -246.
54. Ramachandran, J. & Ullrich, A. (1987). Hormonal regulation of protein tyrosine kinase activity. Trends Pharmacol. Sci. 8: 28-31.
55. Battistini, B., Chailler, P., D'Orléans, J., Brière, N. & Sirois, P. (1993). Growth regulatory properties of endothelins. Peptides 14: 385-399.
56. Pedram, A., Razandi, M., Hu R-M & Levin, E.R. (1997). Vasoactive peptides modulate vascular endothelial cell growth factor production and endothelial cell proliferation and invasion. J. Biol. Chem. 272: 17097-17013.
57. Asham, E.H., Loizidou, M. & Taylor, I. (1998). Endothelin-1 and tumour development. Eur. J. Surg. Oncol. 24: 57-59.
58. Kusuhara, M., Yamaguchi, K., Nagasaki, K., Hayashi, C., Suzaki, A., Nori, S., Hanada, S., Nakamura, Y. and Abe, K. (1990). Production of endothelin in human cancer cell lines. Cancer Res. 50: 3257-3261.
59. Schichiri, M., Hirata, Y., Nakajiroa, T., & o, K., Imal, T., Yanagisawa, M., Mazaki, T. and Marumo, F. (1991). Endothelin-1 is an autocrine/paracrine growth factor for human cancer cell lines. J. Clin. Invest. 87: 1867- 1871.
60. Pagotto, U., Arzberger, T., Hopfer, U., Weidnl, A. & Stella, G. (1995). Cellular localization of endothelin receptor mRNA ET_{A} and ET_{B} in the brain tumour and normal human brain. J. Cardiovasc. Pharmacol. 26 (Suppl. 3): S104-106.
61. Stiles, J.D., Ostrow, P.T., Balos, L.L., Greenberg, S.J., Plunkett, R., Grand, W. & Heffner Jr., R.R. (1997). Correlation of endothelin-1 and transforming growth factor β1 with malignancy and vascularity in human gliomas. J. Neurobiol. 56: 435-439.
62. Ishibashi, M., Fujita, M., Nagai, K., Koko, M., Furue, H., Haku, E., Osamura, Y. & Yamaji, T. (1993). Production and secretion of endothelin by hepatocellular carcinoma. J. Clin. Endocrinol. Metab. 76: 378-383.
63. Giaid, A., Hamid, Q.A., Springall, D.R., Yanagisawa, M., Shinmi, O., Sawamura, T., Masaki, T., Kimura, S., Corrin, B. and Polak, J.M. (1990). Detection of endothelin immunoreactivity and mRNA in pulmonary tumours. J. Pathol. 162: 15-22.
64. Nelson, J., Chan, K., Hedican, S., Magnuson, S., Opgenorth, T., Bova, G. & Simons, J. (1996). Endothelin- 1 production decreased B receptor expression in advanced prostate cancer. Cancer Res. 56: 663-668.
65. Kikuchik, K., Nakagawa, H., Kadono, T., Etoh, T., Byers, H., Mihm, M. & Tamaki, K. (1996). Decreased ETB receptor expression in human metastatic melanoma cells. Biochem. Biophys. Res. Commun. 219: 734-739.
66. Ziche, M., Morbidelli, L., Donnini, S. & Ledda, F. (1995). ETB receptor promote proliferation and migration of endothelial cells. J. Cardiovasc. Pharmacol. 26 (Suppl. 3): S284-S286.
67. Inagaki, H., Bishop, A., Escrig, C., Wharton, J., Allen-Mersh, T. & Polak, J. (1991). Localization of endothelin like immunoreactivity and endothelin binding sites in human colon. Gastroenterology 101: 47-54.
68. Inagaki, H., Bishop, A., Eimoto, T. & Polak, J. (1992). Autoradiographic localization of endothelin-1 binding sites in human colonic cancer tissue. J. Pathol. 168: 263-267.
69. Eguchi, S., Hirata, Y., Imai, T. & Marumo, P. (1995). ET-1 as an autocrine growth factor for endothelial cells. J. Cardiovasc. Pharmacol. 26 (Suppl. 3): S279-S283.
70. Leveson, S., Wiggins, P., Giles, G., Parkin, A. & Robinson, P. (1985). Deranged blood flow patterns in the detection of liver metastases. Br. J. Surg. 72: 128-130.
71. Carter, R., Anderson, J., Cooke, T., Baxter, J. & Angerson, W. (1994). Splanchnic blood flow changes in the presence of hepatic tumours, evidence of humoral mediator. Br. J. Cancer 69: 1025-1026.
72. Nakamura, T., Ebihara, I., Fukui, M., Tomino, Y. & Koide, H. (1995). Effect of a specific endothelin receptor A antagonist on mRNA levels for extracellular matrix components and growth factors in diabetic glomeruli. Diabetes 44: 895-899.
73. Lee, K.M., Tsai, K.Y., Wang, N. & Ingber, D.E. (1998). Extracellular matrix and pulmonary hypertension: control of vascular smooth muscle cell contractility. Am. J. Physiol. 274: H76-82.
74. Hirshfeld, J.W. Jr, Schwartz, J.S., Jugo, R., MacDonald, R.G., Goldberg, S., Savage, M.P., Bass, T.A., Vetrovec, G., Cowley, M., Taussig, A.S., et al. (1991) Restenosis after coronary angioplasty: a multivariate statistical model to relate lesion and procedure variables to restenosis. The M-HEART Investigators. J. Am. Coll. Cardiol. 18: 647-656.
75. Maseri, A. (1996). An unresolved, persisting challenge for percutaneous transluminal coronary angioplasty: how to identify the lesions that will not restenose. Eur. Heart J. 17: 814-815.
76. Kirchengast, M. & Munter, K. (1998). Endothelin and restenosis. Cardiovasc. Res. 39: 550-555.
77. Kramer, B.K., Ackermann, M., Kohler, S.M. & Riegger, G.A. (1994). Role of endothelin in hypertension. Clin. Investig. 72: 88-93.
78. Levin, E. R. (1995). Endothelins: mechanisms of disease. New Eng. J. Med. 333: 356-363.
79. Rohmeiss, P., Birck, R., Braun, C., Kirchengast, M. & van der Woude, F.J. (1999). Targets for endothelin in the diseased kidney: clues for therapeutic intervention. Exp. Nephrol. 7: 1-10.
80. Cardell, L.O., Uddman, R. & Edvinsson, L. (1994). Endothelins: a role in cerebrovascular disease? Cephalalgia 14: 259-65.
81. Cosentino, F. & Katusic, Z. S. (1994). Does endothelin-1 play a role in the pathogenesis of cerebral vasospasm? Stroke 25: 904-908.
82. Battistini, B. & Dussault, P. (1998). The many aspects of endothelins in ischemia-reperfusion injury: emergence of a key mediator. J. Invest. Surg. 11: 297-313.
83. Mauel, J. (1990). Macrophage-parasite interactions in Leishmania infections. J. Leukoc. Biol. 47: 187-193.
84. Belosevic, M., Findbloom, D.S., Meltzer, M.S. & Nacy, C.A. (1990). IL-2. A cofactor for induction of activated macrophage resistance to infection. J. Immunol. 145: 831-839.
85. Olivier, M., Bertrand, S. & Tanner, C. E. (1989). Killing of Leishmania donovani by activated liver macrophages from resistant and susceptible strains mice. Int. J. Parasitol. 19: 377-383 .
86. Skov, C.B. & Twohy, W. (1974). Cellular immunity to Leishmania donovani. I. The effect of T cell depletion on resistance to L. donovani in mice. J. Immunol. 113: 2004-2011.
87. Murray, H.W., Masur, H. & Keithly, J.S. (1982). Cell-mediated immune response in experimental visceral leishmaniasis. I. Correlation between resistance to Leishmania donovani and lymphokine-generating capacity. J. Immunol. 129: 344-350.
88. Olivier, M. & Tanner, C. (1989). The effect of cyclosporin A in murine visceral leishmaniasis. Trop. Med. Parasitol. 40: 32-38.
89. Reiner, N.E. & Finke, J.H. (1983). Interleukin 2 deficiency in murine leishmaniasis donovani and its relationship to depressed spleen cell responsees to phytohemagglutinin. J. Immunol. 131: 1487-1491.
90. Murray, H.W., Stem, J.J., Welte, K., Rubin, B.Y., Carriero, S.M. & Nathan, C.F. (1987). Experimental visceral leishmaniasis: production of interleukin 2 and interferon-gamma, tissue immune reaction, and response to treatment with interleukin 2 and interferon-gamma. J. Immunol. 138: 2290-2297.
91. Olivier, M., Proulx, C. & Tanner, C.E. (1989). Importance of lymphokines in the control of the multiplication and dispersion of Leishmania donovani within liver macrophages of resistant and susceptible mice. J. Parasitol. 75: 720-727.
92. Green, S.P., Hamilton, J.A. & Phillips, W.A. (1992). Zymosan-triggered tyrosine phosphorylation in mouse bone-marrow-derived macrophages is enhanced by respiratory-burst priming agents. Biochem. J. 288: 427-432.
93. Greenberg, S., Chang. P. & Silverstein, S.C. (1993). Tyrosine phosphorylation of the gamma subunit of Fc gamma receptors, p72syk, and paxillin during Fc receptor-mediated phagocytosis in macrophages. J. Biol. Chem. 269: 3897-3902.
94. Dong, Z., Qi, X., Xie, K. & Fidler, I.J. (1993). Protein tyrosine kinase inhibitors decrease induction of nitric oxide synthase activity in lipopolysaccharide-responsive and lipopolysaccharide-nonresponsive murine macrohages. J. Immunol. 151: 2717-2724.
95. Dong, Z., O'Brian, C.A. & Fidler (1993). I.J., Activation of tumoricidal properties in macrophages by lipopolysaccharide requires protein-tyrosine kinase activity. J. Leukoc. Biol. 53: 53-60.
96. Glaser, K.B., Sung, A., Bauer, J. & Weichman, B.M. (1993). Regulation of eicosanoid biosynthesis in the macrophage. Involvement of protein tyrosine phosphorylation and modulation by selective protein tyrosine kinase inhibitors. Biochem. Pharmacol. 45: 711-721.
97. Golden, A., Nemeth, S.P. & Brugge, J.S. (1986). Blood platelets express high levels of the pp60c-src-specific tyrosine kinase activity. Proc. Natl. Acad. Sci. USA 83: 852-856.
98. Golden, A. & Brugge, J.S. (1989). Thrombin treatment induces rapid changes in tyrosine phosphorylation in platelets. Proc. Natl. Acad. Sci. USA 86: 901-905.
99. Walton, K.M. & Dixon, J.E. (1993). Protein tyrosine phosphatases. Annu. Rev. Biochem. 62: 101-120.
100. Fantl, W.J., Johnson, D.E. & Williams, L.T. (1993). Signalling by receptor tyrosine kinases. Annu. Rev. Biochem. 62: 453-481.
101. Chao, W., Liu, H., Hanahan, D.J. & Olson, M.S. (1992). Protein tyrosine phosphorylation and regulation of the receptor for platelet-activating factor in rat Kupffer cells. Effect of sodium vanadate. Biochem. J. 288: 777-784.
102. Secrist, J.P., Bums, L.A., Kamitz, L., Koretsky, G.A. & Abraham, R.T. (1993). Stimulatory effects of the protein tyrosine phosphatase inhibitor, pervanadate, on T-cell activation events. J. Biol. Chem. 268: 5886-5893.
103. Imbert, V., Peyron, J.F., Farahi Far, D., Mari, B., Auberger, P. & Rossi, B. (1994). Induction of tyrosine phosphorylation and T-cell activation by vanadate peroxide, an inhibitor of protein tyrosine phosphatase. Biochem. J. 297: 163-173.
104. Barbeau, B., Bernier, R., Dumais, N., Briand, G., Olivier, M., Faure, R.,. Posner, B.I. & Tremblay, M. (1997). Activation of HIV-1 LTR transcription and virus replication via NF-κB-dependent and -independent pathways by potent phosphotyrosine phosphatase inhibitors, the peroxovanadium compounds. J. Biol. Chem. 272: 12968-12977.
105. Sirois, E., Côté, M.F. & Doillon, C.J. (1993). Growth factors and biological supports for endothelial cell lining: in vitro study. Int. J. Artificial Organs 16: 609-619.
106. Janvier, R., Sourla, A., Koutsilieris, M. & Doillon, C. (1997). Stromal Fibroblasts are required for PC-3 human prostate cancer cells to produce capillary-like formation of endothelial cells in a three-dimensional co-culture system. Anticancer res. 17: 1551-1558.
107. Nicosia, R.F. & Ottineti, A. (1990) Growth of microvessels in serum-free-matrix culture of aorta. A quantitative assay of angiogenesis in vitro. Lab. Invest., 63:115-122.
108. Passaniti, A., Taylor, R.M., Pili, R., Guo, Y., Long, P.V., Haney, J.A., Pauly, R.R., Grant, D.S. & Martin, G.R. (1992). A simple quantitative method for assessing angiogenesis and antiangiogenic agents using reconstituted basement membrane, heparin, and fibroblast growth factor. Lab. Invest. 67 : 519-528.
109. Laporte, S., & Escher, E. (1992). Neointima formation after vascular injury is angiotensin II mediated. Biochem. Biophys. Res. Comm. 187:1510-1516.
110. Lima, G.M., Vallochi, A.L., Silva, U.R., Bevilacqua, E.M., Kiffer, M.M. & Abrahamsohn, I.A. (1998). The role of polymorphonuclear leukocytes in the resistance to cutaneous leishmaniasis. Immunol. Lett. 64: 145-151.
111.Hobbs, A.J., Higgs, A. & Moncada, S. (1999). Inhibition of nitric oxide synthase as a potential therapeutic target. Annu. Rev. Pharmacol. Toxicol. 39: 191-220.
112.Ajuevor, M.N., Virag, L., Flower, R.J., Perretti, M. & Szabo, C. (1998). Role of inducible nitric oxide synthase in the regulation of neutrophil migration in zymosan-induced inflammation. Immunology. 95: 625-630.
113.Yan, X.T., Tumpey, T.M., Kunkel, S.L., Oakes, J.E. & Lausch, R.N. (1998). Role of MIP-2 in neutrophil migration and tissue injury in the herpes simplex virus-1-infected cornea. Invest. Ophthalmol. Vis. Sci. 39: 1854-1862.
114. Duong, M., Ouellet, N., Simard, M., Bergeron, Y., Olivier, M. & Bergeron M.G.B. (1998). Kinetic study of host defense and inflammatory response to Aspergillus fumigatus in steroid-induced immunosuppressed mice. J. Infect. Dis. 178: 1472-1482.
115. Tam, F.W., Karkar, A.M., Smith, J., Yoshimura, T., Steinkasserer, Kurrle, R., Langner, K. & Rees, A.J. (1996) Differential expression of macrophage inflammatory protein-2 and monocyte chemoattractant protein-1 in experimental glomerulonephritis. Kidney Int. 49: 715-721.
116. Stenger, S., Thüring, H., Röllinghoff, M. & Bogdan, C. (1994). Tissue expression of inducible nitric oxide synthase is closely associated with resistance to Leishmania major. J. Exp. Med. 180: 783-793.
117. Stenger, S., Donhauser, N., Thüring, H., Röllinghoff, M. & Bogdan, C. (1996). Reactivation of latent leishmaniasis by inhibition of inducible nitric oxide synthase. J. Exp. Med. 183: 1501-1514.
118. Pani, G., Kozlowski, M., Cambier, J., Mills, G.B. & Siminovitch, K.A. (1995) Identification of the tyrosine phosphatase PTP1C as a B cell antigen receptor-associated protein involved in the regulation of B cell signaling. J. Exp. Med.. 181: 2077-2084.
119. Klingmüller, U., Lorenz, U., Cantley, L.C., Neel, B.G. & Lodish, H.F. (1995). Specific recruitment of SH-PTP1 to the erythropoietin receptor causes inactivation of JAK2 and termination of proliferative signals. Cell 80: 729-738.

## Claims

1. Use of a compound of the following formula: 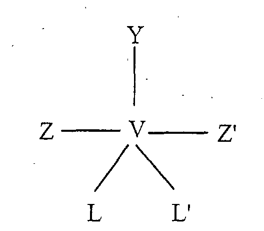 wherein
Y is oxygen or hydroxyl,
Z and Z' are independently selected from oxygen and peroxide and at least one of them is peroxide, and
L or L' are any group which can donate one electron pair, in the preparation of a medicament for treating any condition susceptible of being improved or prevented by inhibition of endothelial cell proliferation in an animal, preferably a human, said condition being selected from the group consisting of
- angiogenesis, said angiogenesis condition being selected from to neovascular glaucoma, retrolental fibroplasia, macular degeneration, neovascularization of corneal grafts, or neovascularization of solid tumors,
- a diabetic complication consisting of vasculopathy or nephropathy, and
- restenosis following angioplasty.

2. Use according to claim 1, wherein the angiogenesis condition is, selected from neovascular glaucoma, retrolental fibroplasia, macular degeneration or neovascularization of corneal grafts.

3. Use according to claim 1, wherein the angiogenesis condition is neovascularization of solid tumors.

4. Use according to claim 1, wherein the condition is the diabetic complication consisting of vasculopathy or nephropathy.

5. Use according to claim 1, wherein the condition is the diabetic complication consisting of vasculopathy.

6. Use according to claim 1, wherein the condition is restenosis following angioplasty.

7. Use of a compound of the following formula: 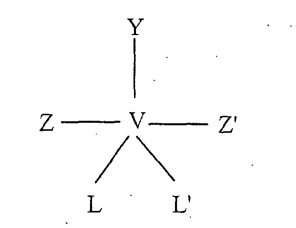 wherein
Y is oxygen or hydroxyl,
Z and Z' are independently selected from oxygen and peroxide and at least one of them is peroxide, and
L or L' are any group which can donate one electron pair,
in the preparation of a medicament for treating at least one diabetic complication selected from the group consisting of vasculopathy and nephropathy, which is susceptible of being improved or prevented by inhibition of an insulin-induced increase in endothelins in an animal, preferably a human.

8. Use of a compound of the following formula: 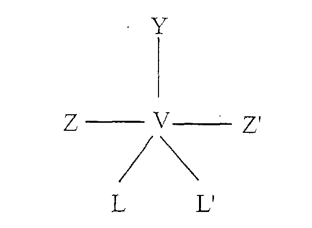 wherein
Y is oxygen or hydroxyl,
Z and Z' are independently selected from oxygen and peroxide and at least one of them is peroxide, and
L or L' are any group which can donate one electron pair,
in the preparation of a medicament for preventing restenosis following angioplasty in an animal, preferably a human.

9. Use according to any one of claims 1 to 8 wherein Y is hydroxyl.

10. Use according to any one of claims 1 to 8 wherein Y is oxygen.

11. Use according to any one of claims 1 to 8 wherein Z is oxygen and Z' is peroxide.

12. Use according to any one of claims 1 to 8 wherein Z and Z' are peroxide.

13. Use according to any one of claims 1 to 8 wherein said compound has the following structure: 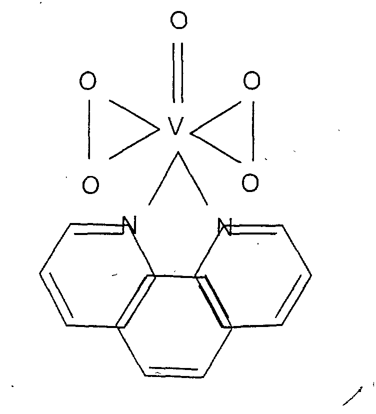 and its potassium salt.

14. Use according to any one of claims 1 to 8 wherein said compound has the following structure: 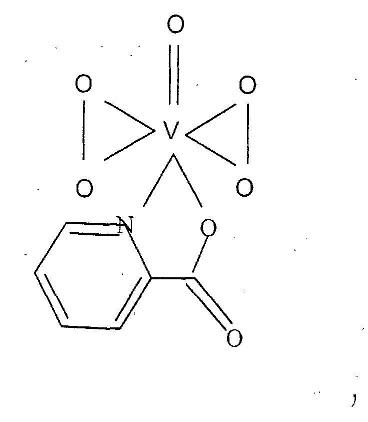 and its potassium salt.

15. Use according to any one of claims 1 to 8 wherein said compound has the following structure: 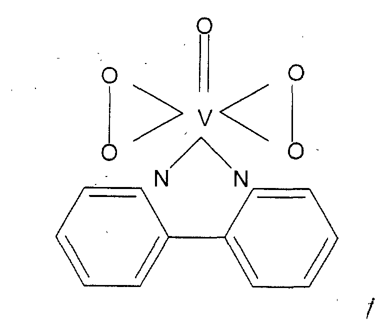 and its potassium salt.

16. Use according to any one of claims 1 to 15, wherein the medicament is capable of achieving an extracellular concentration of about 0.1 to 100 µM.

17. Use according to claim 16, wherein the medicament is capable of achieving an extracellular concentration of obout 2 - 40 µM.

## Patentansprüche

1. Verwendung einer Verbindung der folgenden Formel: 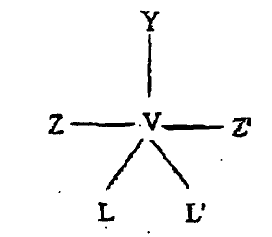 wobei
Y Sauerstoff oder Hydroxyl ist,
Z und Z' unabhängig aus Sauerstoff und Peroxid ausgewählt sind und mindestens eines von diesen Peroxid ist, und
L oder L' ein beliebiger Rest sind, der ein Elektronenpaar spenden kann,
zur Herstellung eines Medikaments zur Behandlung eines Zustandek, der durch Inhibieren der endothelen Zellproliferation in einem Tier, vorzugsweise einem Menschen, verbessert oder vermieden werden kann, wobei der Zustand ausgewählt ist aus
- Angiogenese, wobei der Angiogenesezustand ausgewählt ist aus neovaskulärem Glaukom, retrolentaler Fibroplasie, Makuladegeneration, Neovaskularisation von kornealen Transplantaten oder Neovaskularisation von festen Tumoren,
- einer diabetischen Komplikation, bestehend aus Vaskulopathie oder Nephropathie, und
- auf Angioplastie folgende Restenose.

2. Verwendung gemäß Anspruch 1, wobei der Angiogenesezustand ausgewählt ist aus neovaskulärem Glaukom, retrolentaler Fibroplasie, Makuladegeneration oder Neovaskularisation von kornealen Transplantaten.

3. Verwendung gemäß Anspruch 1, wobei der Angiogenesezustand Neovaskularisation von festen Tumoren ist.

4. Verwendung gemäß Anspruch 1, wobei der Zustand die diabetische Komplikation, bestehend aus Vaskulopathie oder Nephropathie, ist.

5. Verwendung gemäß Anspruch 1, wobei der Zustand die diabetische Komplikation, bestehend aus Vaskulopathie, ist.

6. Verwendung gemäß Anspruch 1, wobei der Zustand auf Angioplastie folgende Restenose ist.

7. Verwendung einer Verbindung der folgenden Formel: 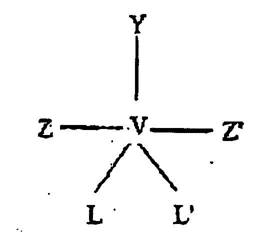 wobei
Y Sauerstoff oder Hydroxyl ist,
Z und Z' unabhängig aus Sauerstoff und Peroxid ausgewählt sind und mindestens eines von diesen Peroxid ist, und
L oder L' ein beliebiger Rest sind, der ein Elektronenpaar spenden kann,
zur Herstellung eines Medikaments zur Behandlung mindestens einer diabetischen Komplikation, ausgewählt aus Vaskulopathie und Nephropathie, die durch Inhibieren eines Insulin-induzierten Anstiegs an Endothelinen in einem Tier, bevorzugt einem Menschen, verbessert oder vermieden werden kann.

8. Verwendung einer Verbindung der folgenden Formel: 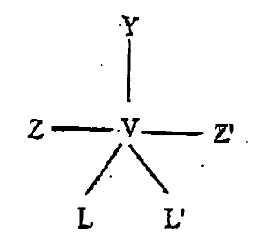 wobei
wobei Y Sauerstoff oder Hydroxyl ist,
Z und Z' unabhängig aus Sauerstoff und Peroxid ausgewählt sind und mindestens eines von diesen Peroxid ist, und
L oder L' ein beliebiger Rest sind, der ein Elektronenpaar spenden kann,
zur Herstellung eines Medikaments zur Vermeidung von auf Angioplastie folgender Restenose bei einem Tier, bevorzugt einem Menschen.

9. Verwendung gemäß einem der Ansprüche 1 bis 8, wobei Y Hydroxyl ist.

10. Verwendung gemäß einem der Ansprüche 1 bis 8, wobei Y Sauerstoff ist.

11. Verwendung gemäß einem der Ansprüche 1 bis 8, wobei Z Sauerstoff ist und Z' Peroxid ist.

12. Verwendung gemäß einem der Ansprüche 1 bis 8, wobei Z und Z' Peroxid sind.

13. Verwendung gemäß einem der Ansprüche 1 bis 8, wobei die Verbindung die folgende Struktur aufweist: 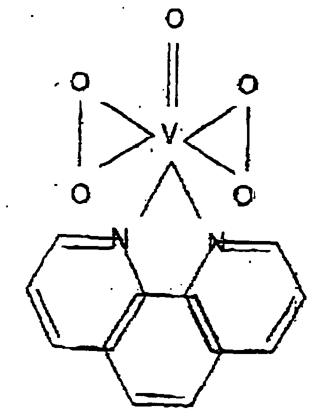 und deren Kaliumsalz.

14. Verwendung gemäß einem der Ansprüche 1 bis 8, wobei die Verbindung die folgende Struktur aufweist: 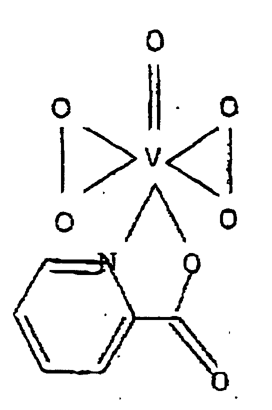 und deren Kaliumsalz.

15. Verwendung gemäß einem der Ansprüche 1 bis 8, wobei die Verbindung die folgende Struktur aufweist: 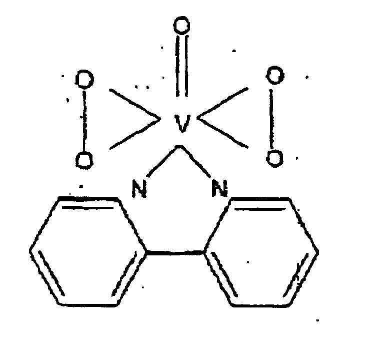 und deren Kaliumsalz.

16. Verwendung gemäß einem der Ansprüche 1 bis 15, wobei das Medikament geeignet ist, eine extrazelluläre Konzentration von etwa 0.1 bis 100 µM zu erreichen.

17. Verwendung gemäß Anspruch 16, wobei das Medikament geeignet ist, eine extrazelluläre Konzentration von etwa 2 bis 40 µM zu erreichen.

## Revendications

1. Utilisation d'un composé de la formule suivante : 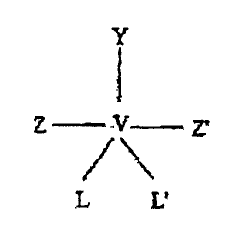 dans laquelle
Y est l'oxygène ou un hydroxyle,
Z et Z' sont indépendamment choisis à partir de l'oxygène et d'un peroxyde et au moins l'un d'entre eux est un peroxyde, et
L ou L' sont un groupe quelconque qui peut donner une paire d'électrons,
dans la préparation d'un médicament destiné à traiter un quelconque état susceptible d'être amélioré ou évité par inhibition de la prolifération des cellules endothéliales chez un animal, préférablement un humain, ledit état étant choisi dans le groupe constitué de
l'angiogenèse, ledit état d'angiogenèse étant choisi parmi un glaucome néovasculaire, une fibroplasie rétrocristallinienne, une dégénération maculaire, une néovascularisation de greffes de cornée, ou une néovascularisation de tumeurs solides,
une complication diabétique consistant en une vasculopathie ou une néphropathie, et
une resténose suivant une angioplastie.

2. Utilisation selon la revendication 1, dans laquelle l'état d'angiogenèse est choisi parmi un glaucome néovasculaire, une fibroplasie rétroctistallinienne, une dégénération maculaire ou une néovascularisation de greffes de cornée.

3. Utilisation selon la revendication 1, dans laquelle l'état d'angiogenèse est une néovascularisation de tumeurs solides.

4. Utilisation selon la revendication 1, dans laquelle l'état d'angiogenèse est la complication diabétique consistant en une vasculopathie ou une néphropathie.

5. Utilisation selon la revendication 1, dans laquelle l'état d'angiogenèse est la complication diabétique consistant en une vasculopathie.

6. Utilisation selon la revendication 1, dans laquelle l'état d'angiogenèse est une resténose suivant une angioplastie.

7. Utilisation d'un composé de la formule suivante : 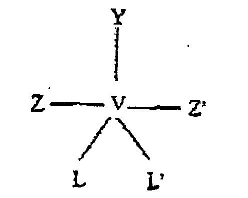 dans laquelle
Y est l'oxygène ou un hydroxyle,
Z et Z' sont indépendamment choisis à partir de l'oxygène et d'un peroxyde et au moins l'un d'entre eux est un peroxyde, et
L ou L' sont un groupe quelconque qui peut donner une paire d'électrons,
dans la préparation d'un médicament destiné à traiter au moins une complication diabétique choisie dans le groupe consistant en une vasculopathie ou une néphropathie, qui est susceptible d'être améliorée ou évitée par inhibition d'une augmentation des endothélines induite par l'insuline chez un animal, préférablement un humain.

8. Utilisation d'un composé de la formule suivante : 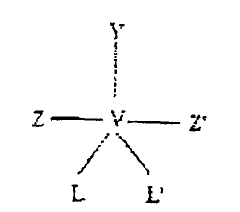 dans laquelle
Y est l'oxygène ou un hydroxyle,
Z et Z' sont indépendamment choisis à partir de l'oxygène et d'un peroxyde et au moins l'un d'entre eux est un peroxyde, et
L ou L' sont un groupe quelconque qui peut donner une paire d'électrons,
dans la préparation d'un médicament destiné à éviter une resténose suivant une angioplastie chez un animal, préférablement un humain.

9. Utilisation selon l'une quelconque des revendications 1 à 8 dans laquelle Y est un hydroxyle.

10. Utilisation selon l'une quelconque des revendications 1 à 8 dans laquelle Y est l'oxygène.

11. Utilisation selon l'une quelconque des revendications 1 à 8 dans laquelle Z est l'oxygène et Z' est un peroxyde.

12. Utilisation selon l'une quelconque des revendications 1 à 8 dans laquelle Z et Z' sont un peroxyde.

13. Utilisation selon l'une quelconque des revendications 1 à 8 dans laquelle ledit composé a la structure suivante : 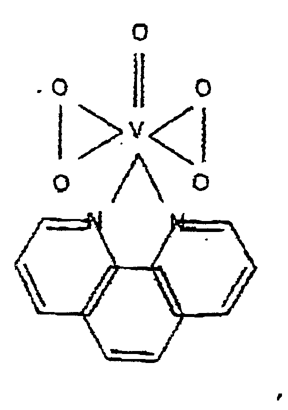 et son sel de potassium.

14. Utilisation selon l'une quelconque des revendications 1 à 8 dans laquelle ledit composé a la structure suivante : 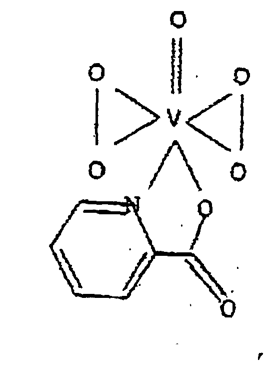 et son sel de potassium.

15. Utilisation selon l'une quelconque des revendications 1 à 8 dans laquelle ledit composé a la structure suivante : 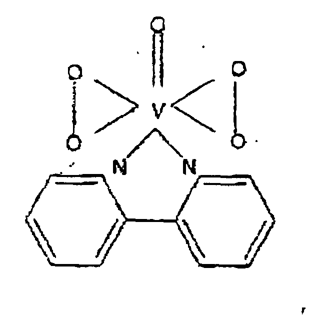 et son sel de potassium.

16. Utilisation selon l'une quelconque des revendications 1 à 15, dans laquelle le médicament est capable d'atteindre une concentration extracellulaire d'environ 0,1 à 100 µM.

17. Utilisation selon la revendication 16, dans laquelle le médicament est capable d'atteindre une concentration extracellulaire d'environ 2 à 40 µM.
